(19) **Europäisches Patentamt / European Patent Office / Office européen des brevets**

(11) **EP 3 060 256 B1**

(12) # EUROPEAN PATENT SPECIFICATION

(45) Date of publication and mention
of the grant of the patent:
**08.05.2019 Bulletin 2019/19**

(21) Application number: **14786915.0**

(22) Date of filing: **21.10.2014**

(51) Int Cl.:
*A61K 31/537* (2006.01)     *C07K 16/30* (2006.01)
*A61K 47/68* (2017.01)     *A61K 9/00* (2006.01)
*A61K 9/19* (2006.01)     *A61K 9/08* (2006.01)
*A61K 47/26* (2006.01)     *A61K 47/18* (2017.01)

(86) International application number:
**PCT/EP2014/072558**

(87) International publication number:
**WO 2015/059147 (30.04.2015 Gazette 2015/17)**

(54) **A NOVEL STABLE FORMULATION**

NEUARTIGE STABILE FORMULIERUNG

NOUVELLE FORMULATION STABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.10.2013 EP 13190276**

(43) Date of publication of application:
**31.08.2016 Bulletin 2016/35**

(73) Proprietor: **Bayer Pharma Aktiengesellschaft
13353 Berlin (DE)**

(72) Inventors:
• **OLBRICH, Carsten
10961 Berlin (DE)**
• **TRILL, Thomas
16552 Schildow (DE)**

(74) Representative: **BIP Patents
c/o Bayer Intellectual Property GmbH
Alfred-Nobel-Straße 10
40789 Monheim am Rhein (DE)**

(56) References cited:
**WO-A1-2010/124797     WO-A2-2007/019232
US-A1- 2004 241 174**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a stable formulation as defined by the claims particularly suitable for the anti-mesothelin immunoconjugate MF-T-SPDB-DM4. The described stable aqueous formulation comprising MF-T-SPDB-DM4 is directly suitable for therapeutic applications and for subsequent lyophilization. The lyophilized powder can be reconstituted with water to create a reconstituted solution which is again suitable for therapeutic applications. It is a further object to provide a stable reconstituted protein formulation which is suitable for therapeutic administrations.

BACKGROUND OF THE INVENTION

Antibody-based therapy is proving very effective in the treatment of various cancers, including solid tumors. Central to the development of a successful antibody-based therapy is the isolation of antibodies against cell-surface proteins found to be preferentially expressed on tumor cells. The mesothelin precursor polypeptide is a glycophosphatidylinositol (GPI)-anchored, glycosylated cell surface protein that is proteolytically cleaved to a 30 kDa N-terminal secreted polypeptide and a 40 kDa, C-terminal polypeptide, which predominantly occurs in the membrane-bound, GPI-anchored form (Chang, K. and I. Pastan, Proc. Natl. Acad. Sei. USA, (1996) 93(1):136), and which is named mesothelin herein. Mesothelin is preferentially expressed by certain tumor cells, particularly mesothelioma cells, pancreatic tumor cells and ovarian carcinoma cells, while its expression is limited in normal tissue, making it an attractive target for the development of tumor therapy (Argani, P. et al., Clin. Cancer Res. (2001) 7(12): 3862; Hassan, R., et a/., Clin. Cancer Res. (2004) 10(12 Pt 1):3937).

Anti-mesothelin antibodies including MF-T, antigen-binding antibody fragments, and variants of these antibodies have been described in WO2009/068204. The described antibodies have special features making them very suitable for a use as immunoconjugates. An immunoconjugate is composed of an antibody specifically recognizing a target cell antigen, such as a tumor cell antigen, and one or several covalently linked molecules of a drug, particularly a cytotoxic drug such as a maytansinoid. Immunoconjugates composed of anti-mesothelin antibodies, antibody fragments, and variants of these antibodies and fragments linked to a chemotherapeutic agent, e.g. maytansinoids, or derivatives thereof have been described in WO2010/124797. A special preferred embodiment of an anti-mesothelin immunoconjugate is MF-T-SPDB-DM4 described in WO2010/124797 in detail.

Unlike traditional organic and inorganic drugs antibody based drugs are larger and more complex. This makes it more difficult to develop formulations which preserve the antibody based drugs in its biologically active form and prevent degradation. Degradation can take place due to chemical instability (resulting in a new chemical entity) or physical instability. The conjugation of drugs, especially cytotoxic drugs, which are often hydrophobic, small molecules, to hydrophilic monoclonal antibodies, introduces additional instability to immunoconjugates. Particle formation in protein pharmaceuticals, in particular, can destabilize the pharmaceutical compound, thus making the formulation less potent or even harmful for clinical use. For example, particles in injected pharmaceutical formulations can cause significant injury in patients. In addition, formation of aggregates is a major degradation pathway of protein pharmaceuticals (Chari et al., Pharm Res. 20, 1325-1336 (2003)), and may lead to undesirable effects such as immunogenicity. Chemical instability of immunoconjugates can result in the generation of free cytotoxic drugs, which can lead to toxic side effects.

A suitable formulation for an immunoconjugate prevents chemical and physical instability over a long time period. Suitable stable liquid or lyophilized formulations for maytansinoid containing immunoconjugates have been described for example in WO2004/004639, WO2004/110498, and WO2007/019232. WO2007/019232 describes a liquid immunoconjugate formulation comprising several excipients, wherein the formulation is a buffered aqueous solution. WO2004/110498 provides a liquid and a lyophilized composition comprising an antibody chemically coupled to a maytansinoid. WO2004/004639 describes suitable formulations for the immunoconjugate huC242-DM1.

Nevertheless the general message from all of these publications is that each immunoconjugate, here MF-T-SPDB-DM4, is a special combination of an antibody, linker and a cytotoxic drug. This combination results in certain physicochemical properties which need an unpredictable solution for a suitable formulation which is provided in this invention.

BRIEF SUMMARY OF THE INVENTION

The invention provides a formulation / composition suitable for therapeutic applications comprising an immunoconjugate formulation comprising: a. 5 mg/ml MF-T-SPDB-DM4, b. 10mM L-Histidine, c. 130mM Glycine, d. 5% sucrose, and e. 0.01 % polysorbate 80 wherein the formulation is a buffered aqueous solution having a pH of 5.5.

The invention also provides a lyophilized composition comprising an immunoconjugate formulation comprising: a. 5 mg/ml MF-T-SPDB-DM4, b. 10mM L-Histidine, c. 130mM Glycine, d. 5% sucrose, and e. 0.01 % polysorbate 80 wherein the composition has a pH of 5.5 when reconstituted with water. The reconstituted solution of this lyophilized composition is suitable for therapeutic applications.

DETAILED DESCRIPTION OF THE INVENTION

This invention relates to the pharmaceutically suitable formulation of the immunoconjugate MF-T-SPDB-DM4 wherein the formulation comprises: a. 5 mg/ ml MF-T-SPDB-DM4, b. 10mM L-Histidine, c. 130mM Glycine, d. 5% sucrose, and e. 0.01 % polysorbate 80 wherein the formulation is a buffered aqueous solution having a pH of 5.5. The invention is also related to lyophilized composition comprising an immunoconjugate formulation comprising: a. 5 mg/ml MF-T-SPDB-DM4, b. 10mM L-Histidine, c. 130mM Glycine, d. 5% sucrose, and e. 0.01 % polysorbate 80 wherein the composition has a pH of 5.5 when reconstituted with water.

The immunoconjugate MF-T-SPDB-DM4 is known in the art (WO2010/124797). MF-T-SPDB-DM4 is an immunoconjugate comprising the antibody MF-T chemically coupled to a maytansinoid (described in WO2009/068204).

Degradation of immunoconjugates, here MF-T-SPDB-DM4, is an undesired effect for pharmaceutical applications. The efficacy or availability of the drug can change dramatically. Degradation can take place due to chemical instability (resulting in a new chemical entity) or physical instability. Chemical instability can result from e.g. deamidation, hydrolysis, oxidation or disulfide exchange. Physical instability can result from e.g. aggregation or adsorption. The conjugation of drugs, especially cytotoxic drugs, which are often hydrophobic, small molecules, to hydrophilic monoclonal antibodies, introduces additional instability to immunoconjugates. Particle formation in protein pharmaceuticals, in particular, can destabilize the pharmaceutical compound, thus making the formulation less potent or even harmful for clinical use. For example, particles in injected pharmaceutical formulations can cause significant injury in patients. In addition, formation of aggregates is a major degradation pathway of protein, and may lead to undesirable effects such as immunogenicity.

So it is an object of this invention to provide pharmaceutically suitable formulations of the immunoconjugate MF-T-SPDB-DM4 which prevent the formation of "high molecular weight aggregates". The term "high molecular weight aggregates" or "HMW", as used herein, refers to aggregates comprising two or more immunoconjugate molecules.

It is a further object of this invention to provide pharmaceutically suitable formulations of the immunoconjugate MF-T-SPDB-DM4 which prevent chemical instability. Chemical instability of immunoconjugates can result in the generation of free cytotoxic drugs (here free DM4 maytansinoid species), which may lead to toxic side effects. So in addition the presence or generation of any kind of "low molecular weight" (LMW) fragments should be minimized or avoided.

The present invention is based on the finding that a composition containing MF-T-SPDB-DM4 was achieved, which allow for a long shelf live as liquid formulation as well as lyophilized composition. This formulation allows for long term storage as lyophilized composition (long shelf live) and after reconstitution for the possibility to store the not used portion over a long time period as aqueous solution.

A typical shelf life for the immunoconjugate compositions of the present invention is about 1 to 5 years, preferably 1 to 4 years, more preferably 2 to 4 years, at 4°C.

This kind of formulations could be achieved by inclusion of excipients that inhibit or reduce aggregation and particle formation. Formulations to stabilize immunoconjugates are known in the art. Suitable stable liquid or lyophilized formulations for maytansinoid containing immunoconjugates have been described for example in WO2004/004639, WO2004/110498, and WO2007/019232. WO2007/019232 describes a liquid immunoconjugate formulation comprising: an immunoconjugate and one or more excipients selected from the group consisting of: sucrose, polysorbate 20, polysorbate 80, cyclodextrin, dextrose, glycerol, polyethylene glycol, mannitol, sodium chloride, and an amino acid, wherein the formulation is a buffered aqueous solution having a pH of 4.5 to 7.6. WO2004/110498 provides a liquid and a lyophilized composition comprising an antibody chemically coupled to a maytansinoid. WO2004/004639 describes suitable formulations for the immunoconjugate huC242-DM1. But it is also well known that each immunoconjugate, here MF-T-SPDB-DM4, is a special combination of an antibody, linker and a cytotoxic drug, resulting in unpredictable formulation problems.

The present invention also relates to the administration of pharmaceutical compositions. Such administration is accomplished orally or parenterally. For immunoconjugates parenteral routes of administration are more preferred. Methods of parenteral delivery include topical, intra-arterial (directly to the tumor), intramuscular, subcutaneous, intramedullary, intrathecal, intraventricular, intravenous, intraperitoneal, or intranasal administration. In addition to the active ingredients, these pharmaceutical compositions may contain suitable pharmaceutically acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Ed. Maack Publishing Co, Easton, Pa.).

Formulations of the invention may be administered using an injector, a pump, a syringe, or any other devices known in the art as well as by gravity. A needle or a catheter may be used for introducing the formulations of the present invention into the body of a patient via certain parenteral routes.

Pharmaceutical compositions suitable for use in the present invention include compositions wherein the active ingredients are contained in an effective amount to achieve the intended purpose, i.e. treatment of a particular disease. The determination of an effective dose is well within the capability of those skilled in the art.

For any compound, the therapeutically effective dose can be estimated initially either in cell culture assays, e.g., neoplastic cells, or in animal models, usually mice, rabbits, dogs, pigs or monkeys. The animal model is also used to achieve a desirable concentration range and route of administration. Such information can then be used to determine useful doses and routes for administration in humans.

The compositions of this invention are formulated to be acceptable in a therapeutic application. "Therapeutic application" refers to treatments involving administration to a subject in need of treatment a therapeutically effective amount of the immunoconjugate MF-T-SPDB-DM4. A "therapeutically effective amount" hereby is defined as the amount of the immunoconjugate MF-T-SPDB-DM4 that is of sufficient quantity to reduce proliferation of mesothelin positive cell or to reduce size of a mesothelin expressing tumor in a treated area of a subject - either as a single dose or according to a multiple dose regimen, alone or in combination with other agents, which leads to the alleviation of an adverse condition, yet which amount is toxicologically tolerable. The subject may be a human or non-human animal (e.g., rabbit, rat, mouse, dog, monkey or other lower-order primate).

The exact dosage is chosen by the individual physician in view of the patient to be treated. Dosage and administration are adjusted to provide sufficient levels of the active moiety or to maintain the desired effect. Additional factors that may be taken into account include the severity of the disease state, e.g., tumor size and location; age, weight and gender of the patient; diet, time and frequency of administration, drug combination(s), reaction sensitivities, and tolerance/response to therapy. Long acting pharmaceutical compositions might be administered for example every 3 to 4 days, every week, once every two weeks, or once every three weeks, depending on half-life and clearance rate of the particular formulation.

The term "pharmaceutical formulation" or "formulation" refers to a preparation which is in such form as to permit the biological activity of an active ingredient contained therein to be effective, and which contains no additional components which are unacceptably toxic to a subject to which the formulation would be administered.

The present invention also provides for a lyophilized powder of the liquid formulation. "Lyophilized" means that the composition has been freeze-dried under vacuum. During lyophilization the liquid formulation is frozen and the solutes are separated from the solvent. The solvent is removed by sublimation (i.e., primary drying) and next by desorption (i.e., secondary drying). Lyophilization results in a cake or powder which can be stored over a long time period. Prior to administration the lyophilized composition is reconstituted in solvent, preferentially sterile water for injection. The term "reconstituted formulation", as used herein, refers to such a lyophilized composition after solubilization.

Lyophilization methods are well known in the art (e.g. Wang, W., Int. J . Pharm., 203, 1-60 (2000)). The inventive lyophilized composition was achieved with two different lyophilization protocols as described in the examples in detail.

In order to prevent degradation during the lyophilization process the inventive liquid composition comprises a cryoprotectant. The term "cryoprotectant", as used herein, refers to an excipient that protects unstable molecules during freezing. The liquid composition prior lyophilization comprises 5% sucrose.

The lyophilized composition can further contain a bulking agent, preferably a crystallizable bulking agent. Bulking agents typically are used in the art to provide structure and weight to the "cake" produced as a result of lyophilization. Any suitable bulking agent known in the art may be used in connection with the inventive lyophilized composition. Suitable bulking agents include, for example, mannitol, dextran, and glycine.

The inventive composition comprises a surfactant. The surfactant is polysorbate 80 at a concentration of 0.01%.

The term "buffer", as used herein, refers to a buffered solution, which pH changes only marginally after addition of acidic or basic substances. The "buffering system" comprises 10mM L-Histidine and 130mM Glycine.

An embodiment of the invention is an immunoconjugate formulation comprising MF-T-SPDB-DM4 as defined by the claims wherein the composition has a pH 5.5.

The invention is an immunoconjugate formulation comprising 5 mg/ml MF-T-SPDB-DM4, as defined by the claims.

The invention is an immunoconjugate formulation comprising MF-T-SPDB-DM4 and a buffering system comprising 10 mM L-Histidine and 130 mM Glycine, as defined by the claims.

The invention is an immunoconjugate formulation comprising MF-T-SPDB-DM4, a buffering system, and a cryoprotectant, which is 5% sucrose in a liquid composition or the liquid composition prior to lyophilization, as defined by the claims.

An embodiment of the invention is an immunoconjugate formulation comprising MF-T-SPDB-DM4, a buffering system, a cryoprotectant, as defined by the claims and a surfactant which is 0.01% polysorbate 80.

The invention is an immunoconjugate formulation comprising 5 mg/ml MF-T-SPDB-DM4, 10 mM L-Histidine, 130 mM Glycine, 5% sucrose, and 0.01% polysorbate 80, at a pH of 5.5.

An embodiment of the invention is a lyophilized composition obtained by lyophilization of the liquid immunoconjugate formulation according to this invention, or obtainable by lyophilization of a liquid immunoconjugate formulation according to this invention.

A preferred embodiment of the invention is a lyophilized composition obtained by freeze-drying of a liquid immunoconjugate formulation according to this invention.

A preferred embodiment of the invention is a lyophilized composition obtained by freeze-drying of a liquid immunoconjugate formulation according to this invention using methods explained in example 6.

A highly preferred embodiment of the invention is a lyophilized composition obtained by freeze-drying of the liquid immunoconjugate formulation comprising 5 mg/ml MF-T-SPDB-DM4, 10 mM L-Histidine, 130 mM Glycine, 5% sucrose, and 0.01% polysorbate 80, at a pH of 5.5, or obtainable by freeze-drying of the liquid immunoconjugate formulation comprising 5 mg/ml MF-T-SPDB-DM4, 10 mM L-Histidine, 130 mM Glycine, 5% sucrose, and 0.01% polysorbate 80 at a pH of 5.5.

An embodiment of this invention is a liquid immunoconjugate formulation obtained by reconstitution of a lyophilized composition according to this invention (called reconstituted formulation). In a preferred embodiment of this invention the lyophilized composition is reconstituted in water, preferentially in sterile water for injection.

Thus, in accordance with the invention, the contents of a lyophilized composition that is to be reconstituted to contain 5 mg/ml MF-T-SPDB-DM4 comprises 0.31 mg L-Histidine, 1.95 mg Glycine, 9.99 mg sucrose, and 0.02 mg polysorbate 80 per mg of the immunoconjugate MF-T-SPDB-DM4. Once reconstituted with water, such a lyophilized composition has a pH of about 5.5.

If not otherwise stated all % values in this case are % weight per volume.

The present invention is further described by the following examples, which are illustrative of the process and should not be construed as limiting the invention. The process parameters given below can be adopted and adapted by the skilled person to suit the particular need.

EXAMPLE 1

This example shows how different liquid compositions containing MF-T-SPDB-DM4 were produced. These compositions were subsequently analyzed as described in the following examples.

The immunoconjugate MF-T-SPDB-DM4 was prepared as described in WO2010/124797. To study several formulation compositions the solution comprising the immunoconjugate MF-T-SPDB-DM4 must be changed in a defined way. Solutions comprising MF-T-SPDB-DM4 were injected onto a preparative size exclusion column filled with Sephadex G25 connected to an ÄKTA explorer (GE Healthcare). Applied solutions were eluted with the final buffer solution of interest. The size of the column enabled a complete buffer exchange so that the collected elution fraction consisted of MF-T-SPDB-DM4 in the buffer solution of interest. The protein concentration was adjusted via ultrafiltration using a Vivapore Cell (10/20, 7500 MWCO; Sartorius).

EXAMPLE 2

This example (not falling under the scope of the claims) shows the effect of several buffer systems on aggregation assessed by visual appearance as well as by dynamic light scattering (DLS) and on immunoconjugate stability measured with differential scanning calorimetry (DSC) for MF-T-SPDB-DM4.

Buffer ingredients as well as the pH have a strong influence to inhibit or to reduce the formation of visible and sub-visible particles. Visible particles can be observed by visual inspection. Sub-visible aggregation is detectable via dynamic light scattering (DLS).

A higher melting temperature $T_m$ measured with DSC is a strong indication for increased immunoconjugate stability.

The MF-T-SPDB-DM4 conjugate was formulated at approximately 5.0 mg/ml in:

(1) 100 mM potassium phosphate pH 7.5
(2) 10 mM L-Histidine, 130 mM Glycine pH 7.3
(3) 20 mM sodium citrate, 10% trehalose pH 6.6
(4) 10 mM L-Histidine, 130 mM Glycine pH 5.5
(5) 0.9% NaCl

**Table 1:** Influence of several buffer systems on aggregation behavior and stability

| Composition | 1 | 2 | 3 | 4 | 5 |
|---|---|---|---|---|---|
| Buffer | KPi | His-Gly | Citrate | His-Gly | - |

(continued)

| Composition | 1 | 2 | 3 | 4 | 5 |
|---|---|---|---|---|---|
| pH | 7.5 | 7.3 | 6.6 | 5.5 | - |
| Visual inspection | ok | ok | ok | ok | ok |
| DLS $d_H$ [nm] | 20 | 19 | 19 | 18 | 20 |
| DSC $T_{m1}$ [°C] | 70.1 | 73.6 | 70.9 | 73.4 | 69.0 |

The buffering system has a strong influence on the immunoconjugate stability indicated by the melting temperature $T_{m1}$, whilst the particle formation and aggregation was not affected. Preferred for MF-T-SPDB-DM4 are as depicted in Table 1 buffering systems containing L-Histidine and Glycine showing an increased $T_{m1}$ of approximately 3°C.

EXAMPLE 3

This example (not falling under the scope of the claims) shows the effect of several buffer systems on aggregation assessed by visual appearance, by dynamic light scattering (DLS), and by size exclusion chromatography (SEC) in the following three experiments:

a. 48 hours storing experiment at 20°C
b. Shaking stress test for 24 hours at 20°C
c. Inverted shaking stress test with stopper contact for 24 hours at 20°C

These experiments try to assess the immunoconjugate stability at non optimal treatment (storage) conditions.
The MF-T-SPDB-DM4 conjugate was formulated at approximately 5.0 mg/ml in:

(1) 100 mM potassium phosphate pH 7.5
(2) 10 mM L-Histidine, 130 mM Glycine pH 7.3
(3) 20 mM sodium citrate, 10% trehalose pH 6.6
(4) 10 mM L-Histidine, 130 mM Glycine pH 5.5
(5) 0.9% NaCl

**Table 2:** Influence of several buffer systems on the aggregation behavior at non optimal treatment conditions.

| Composition | | 1 | 2 | 3 | 4 | 5 |
|---|---|---|---|---|---|---|
| Buffer | | KPi | His-Gly | Citrate | His-Gly | - |
| pH | | 7.5 | 7.3 | 6.6 | 5.5 | - |
| **48h/20°C Storing test** | visual | ok | ok | ok | ok | ok |
| | DLS $d_H$ [nm] | 22 | 20 | 14 | 16 | 16 |
| | $c_{Ab}$ [mg/ml] | 5.7 | 3.9 | 4.9 | 4.5 | 5.0 |
| | $c_{Ab}$ [%] | 102.5 | 102.7 | 102.5 | 99.8 | 99.2 |
| | DM4/Ab Ratio | 2,9 | 2,9 | 2,9 | 2,7 | 2,9 |
| | Monomer [%] | 92.8 | 95.8 | 93.3 | 95.2 | *83.3* |
| | LMW1+2 [%] | 0.4 | 0.4 | 1.6 | 0.6 | *11.8* |
| | HMW Dimer [%] | 6.8 | 3.8 | *5.1* | 4.2 | 4.8 |
| **Shaking test** | visual | *aggregates* | ok | ok | ok | ok |
| | DLS $d_H$ [nm] | 12 | 14 | 12 | 18 | 17 |
| | $c_{Ab}$ [mg/ml] | *4.0* | 4.0 | 5.0 | 4.7 | 5.1 |
| | $c_{Ab}$ [%] | *72.5* | 105.1 | 104.6 | 103.8 | 102.8 |
| | DM4/Ab Ratio | 2,6 | 2,8 | 2,9 | 2,6 | 2,9 |
| | Monomer [%] | - | 95.5 | 93.3 | 95.1 | *83.0* |
| | LMW 1+2 [%] | - | 0.7 | 1.6 | 0.7 | *12.3* |
| | HMW Dimer [%] | - | 3.8 | *5.1* | 4.2 | 4.8 |

(continued)

| Composition | | 1 | 2 | 3 | 4 | 5 |
|---|---|---|---|---|---|---|
| **Inv.shaking (stopper contact)** | visual | *aggregates* | ok | ok | ok | ok |
| | DLS $d_H$ [nm] | 17 | 11 | 12 | 13 | 22 |
| | $c_{Ab}$ [mg/ml] | *4.3* | *3.5* | 4.8 | 4.5 | 5.0 |
| | $c_{Ab}$ [%] | *77.2* | *92.0* | 101.5 | 99.6 | 99.6 |
| | DM4/Ab Ratio | 2,6 | 2,8 | 2,9 | 2,7 | 2,9 |
| | Monomer [%] | - | 95.7 | 93.0 | 95.0 | *82.7* |
| | LMW1+2 [%] | - | 0.6 | 1.6 | 0.5 | *12.3* |
| | HMW Dimer [%] | - | 3.8 | *5.4* | 4.1 | 5.0 |

The buffering conditions have a strong effect on the aggregation behavior at non optimal treatment (storage) conditions, which was not obvious in example 2. In example 2 it could only be observed that the buffering systems containing L-Histidine and Glycine show an increased $T_{m1}$ indicating higher immunoconjugate stability.

As shown in Table 2 composition 1 and composition 5 have a strong behavior to form aggregates. For composition 1 the tendency to aggregate is already obvious by visual inspection in the shaking test as well as in the inverted shaking experiment. For composition 5 size exclusion chromatography (SEC) results show a dramatic immunoconjugate monomer decrease indicating aggregation. Composition 3 shows an increase of immunoconjugate dimer formation and therefore reveals the tendency for aggregation.

Surprisingly the composition 2 containing L-Histidine and Glycine at pH 7.3 shows a loss of protein concentration in the inverted shaking (stopper contact) experiment. The relation of protein concentration before and after the shaking experiment (cAb [%]) is only 92% compared to 99.6% at pH 5.5. This loss of protein might be due to an increased adsorption to the stopper at pH 7.3 compared to pH 5.5. This is even more surprising as a decrease of immunoconjugate stability assed by DSC and an increased aggregation behavior could not be observed.

This experiment shows that compositions for MF-T-SPDB-DM4 containing L-Histidine and Glycine are preferred and compositions for MF-T-SPDB-DM4 containing L-Histidine and Glycine at pH 5.5 are highly preferred.

EXAMPLE 4

This example shows the effect of polysorbate 80 on protein aggregation assessed by visual inspection and DLS.

The MF-T-SPDB-DM4 conjugate was formulated at approximately 5.0 mg/ml in:

10 mM L-Histidine, 130 mM Glycine pH 5.5, containing 0.0 to 0.1 % polysorbate 80 (only 0.01% falling inside the scope of the claims)

**Table 3**: Influence of polysorbate 80 on protein aggregation

| Conditions | polysorbate 80 [%] | Visual inspection | DLS $d_H$ [nm] | Span$_{25-75}$ | $C_{Ab}$ [mg/ml] |
|---|---|---|---|---|---|
| Without agitation | 0.000 | clear | 8 | 0.41 | 5.09 |
| | 0.001 | clear | 17 | 0.38 | 5.11 |
| | 0.005 | clear | 15 | 0.45 | 5.09 |
| | 0.010 | clear | 8 | 0.25 | 5.09 |
| | 0.100 | clear | 17 | 0.37 | 4.61 |
| Inv. shaking (stopper contact) | 0.000 | clear | 17 | 0.39 | 5.04 |
| | 0.001 | clear | 16 | 0.45 | 5.19 |
| | 0.005 | clear | 18 | 0.45 | 5.06 |
| | 0.010 | clear | 19 | 0.33 | 4.93 |
| | 0.100 | clear | 22 | 0.39 | 4.62 |

The addition of polysorbate 80 does not have any negative effect in the preferred puffer system up to a concentration of 0.1% (see Table 3). The addition of a detergent is generally favored in order to avoid uncontrolled adsorption during storage and application. A polysorbate 80 concentration of about 0.01% is preferred.

EXAMPLE 5

This example shows the stability of preferred liquid formulations over a 14 day time period. In addition two sucrose concentrations were checked for protein aggregation assessed by visual inspection, DLS and size exclusion chromatography (SEC).

Sucrose is a very powerful and one of the commonly used lyoprotectants. For a formulation suitable for liquid storage as well as lyophilization the influence of sucrose on the MF-T-SPDB-DM4 immunoconjugate was assessed.

The MF-T-SPDB-DM4 conjugate was formulated at approximately 5.0 mg/ml in:

(1) 10% sucrose, 0.01% polysorbate 80, 10 mM L-Histidine, 130 mM Glycine pH 5.5 (not according to the invention)
(2) 5% sucrose, 0.01% polysorbate 80, 10 mM L-Histidine, 130 mM Glycine pH 5.5

**Table 4:** Influence of sucrose on protein aggregation

| Composition | | Time [d] | Storage temperature | Visual inspection | DLS $d_H$ [nm] | $C_{Ab}$ [mg/ml] | SEC HMW [area%] | SEC Monomer [area%] | SEC LMW [area%] |
|---|---|---|---|---|---|---|---|---|---|
| 1 | 10% sucrose | start | - | clear | 15 | 4.99 | 4.32 | 95.64 | 0.04 |
| | | 14 | 2 - 8°C | clear | 22.5 | 4.93 | 4.28 | 95.45 | 0.27 |
| | | 14 | 25°C | clear | 20.2 | 4.98 | 3.79 | 95.80 | 0.40 |
| | | 14 | 40°C | clear | 17.7 | 5.05 | 6.12 | 93.1 | 0.78 |
| 2 | 5% sucrose | start | - | clear | 19 | 4.93 | 3.6 | 96.4 | 0.0 |
| | | 14 | 2 - 8°C | clear | 20 | 5.2 | 3.7 | 96.1 | 0.2 |
| | | 14 | 25°C | clear | 22 | 5.33 | 3.9 | 95.7 | 0.4 |
| | | 14 | 40°C | clear | 19 | 5.25 | 5.3 | 93.8 | 0.9 |

Over a 14 day time period compositions containing the MF-T-SPDB-DM4 immunoconjugate are stable. These are compositions suitable for lyophilization as shown in example 6 and example 8. No aggregation could be observed by visual inspection, DLS and SEC. The monomer content is very stable. Even at elevated temperatures of 40°C the liquid formulation does not show aggregation over a 14 day time period.

Compositions containing 5 to 10% of sucrose are preferred formulations. Compositions containing 5% of sucrose are highly preferred due to a lower tonicity.

This experiment also shows that a reconstituted solution after lyophilization can be stored over a long time period without aggregation.

EXAMPLE 6

This example shows the robustness of lyophilization and the suitability of the liquid composition for lyophilization. In addition the results achieved after reconstitution of the lyophilized samples clearly show that the composition in addition allows for a lyophilized formulation.

The MF-T-SPDB-DM4 conjugate was formulated at 5.0 mg/ml in the highly preferred embodiment:
10 mM L-Histidine, 130 mM Glycine, 5% sucrose, 0.01% polysorbate 80, pH 5.5

Two lyophilization methods have been used: conditions 1: a quite harsh method taking in total approximately 62.5 hours; conditions 2: a more gentle method taking in total approximately 95.5 hours. The details of the lyophilization cycles have been summarized in Table 5.

**Table 5:** Parameters of the used lyophilization cycles

|  |  | Conditions 1 | | | Conditions 2 | | |
|---|---|---|---|---|---|---|---|
|  |  | Time [hh:mm] | Temp [°C] | Pressure [mbar] | Time [hh:mm] | Temp [°C] | Pressure [mbar] |
|  | Loading | 00:01 | 5.0 |  | 00:01 | 20.0 |  |
|  | Freezing | 02:00 | -50.0 |  | 00:30 | -5.0 |  |
|  | Freezing |  |  |  | 01:00 | -5.0 |  |
|  | Freezing |  |  |  | 01:00 | -45.0 |  |
|  | Freezing |  |  |  | 03:30 | -45.0 |  |
|  | Evacuation | 00:30 | -50.0 | 0.130 | 00:30 | -45.0 | 0.100 |
|  | Prim. drying | 02:00 | -50.0 | 0.130 | 00:01 | -45.0 | 0.100 |
|  | Prim. drying | 00:30 | -50.0 | 0.066 | 01:00 | -10.0 | 0.100 |
|  | Prim. drying | 02:00 | 0 | 0.066 | 75:00 | -10.0 | 0.100 |
|  | Prim. drying | 16:40 | 0 | 0.066 |  |  |  |
|  | Prim. drying | 01:30 | 0 | 0.053 |  |  |  |
|  | Sec. drying | 03:00 | 30.0 | 0.053 | 01:00 | 40.0 | 0.050 |
|  | Sec. drying | 36:40 | 30.0 | 0.053 | 12:00 | 40.0 | 0.050 |
|  |  |  |  |  |  |  |  |
| Summary | Loading | 00:01 | | | 00:01 | | |
|  | Freezing | 02:00 | | | 06:30 | | |
|  | Primary drying | 20:40 | | | 76:01 | | |
|  | Secondary drying | 39:40 | | | 13:00 | | |
|  | **Total** | **62:21** | | | **95:32** | | |

The lyophilization methods described above result in a white cake or powder, which can be reconstituted rapidly (around 1 min) in water. If reconstituted at the same protein concentration as before lyophilization (5 mg/ml) a clear solution without any particles was observed. No aggregation or hints of aggregation were detected (see Table 6). The SEC monomer content is in the same range as with no lyophilization (see Table 4 for comparison).

In depth analysis of the reconstituted solution included detection of non-visible particles via HIAC method, in addition the more sensitive MFI (micro flow imaging) method was deployed. The particles / container (volume 12.5 ml) are well within generally accepted criteria. This is an important requirement for the suitability as a pharmaceutical product.

In Table 6 properties of the samples after reconstitution with water for injection have been summarized.

**Table 6:** Characterization of lyophilized batches after reconstitution with water [final volume 12.5 ml / container]

| Batch | **Batch 1** | **Batch 2** | **Batch 3** |
|---|---|---|---|
| Lyophilization | Conditions 1 | Conditions 2 | Conditions 2 |
| Reconstitution time | 62 sec | 41 sec | 33 sec |
| Visual Inspection | clear | clear | clear |
| SEC, Monomer [area%] | 92.4 | 92.2 | 91.8 |
| SEC, LMW, [area%] | 1.0 | 1.4 | 1.4 |
| pH | 5.5 | 5.7 | 5.7 |
| Protein concentration | 5.0 mg/ml | 5.0 mg/ml | 4.9 mg/ml |
|  |  |  |  |
|  | particles/ container | particles/ container | particles/ container |

(continued)

| Batch | Batch 1 | Batch 2 | Batch 3 |
|---|---|---|---|
| Non visible particles HIAC method. ≥ 2µm | 1812 | 734 | 520 |
| Non visible particles. HIAC method. ≥ 5µm | 340 | 173 | 115 |
| Non visible particles. HIAC method. ≥ 10µm | 90 | 38 | 38 |
| Non visible particles. HIAC method. ≥ 50µm | 9 | 4 | 1 |
| Non visible particles. MFI method. ≥ 2µm | 3185 | 2137 | 2258 |
| Non visible particles. MFI method. ≥ 5µm | 540 | 192 | 359 |
| Non visible particles. MFI method. ≥ 10µm | 148 | 55 | 71 |
| Non visible particles. MFI method. ≥ 25µm | 28 | 9 | 0 |

EXAMPLE 7

This example shows the chemical stability of the immunoconjugate MF-T-SPDB-DM4 in several preferred liquid formulations over a 14 day time period. Two different sucrose concentrations were used.

The MF-T-SPDB-DM4 conjugate was formulated at approximately 5.0 mg/ml in:

(1) 10% sucrose, 0.01% polysorbate 80, 10 mM L-Histidine, 130 mM Glycine pH 5.5 (not according to the invention)

(2) 5% sucrose, 0.01% polysorbate 80, 10 mM L-Histidine, 130 mM Glycine pH 5.5

MF-T-SPDB-DM4 is stable at different storage conditions over 14 days long time period (see Table 7). Even at elevated temperatures of 25°C or 40°C only small amounts of free toxophor species (free DM4-linker, free DM4) are detectable. So the preferred compositions are physically as well as chemically stable

**Table 7:** Chemical stability of immunoconjugate MF-T-SPDB-DM4 (composition 1 not according to the invention) ~~in preferred formulations~~

| Composition | | Time [d] | Storage temperature | Visual inspection | C_Ab [mg/ ml] | DM4/Ab ratio | free DM4-linker [µg/ ml] | free DM4 [µg/ ml] |
|---|---|---|---|---|---|---|---|---|
| 1 | 10% sucrose | start | - | clear | 4.99 | 2.7 | - | - |
| | | 14 | 2 - 8°C | clear | 4.93 | 2.7 | 0.003 | <0.002 |
| | | 14 | 25°C | clear | 4.98 | 2.7 | 0.106 | <0.002 |
| | | 14 | 40°C | clear | 5.05 | 2.4 | 0.349 | 0.004 |
| 2 | 5% sucrose | start | - | clear | 4.93 | 2.4 | - | - |
| | | 14 | 2 - 8°C | clear | 5.2 | 2.8 | 0.021 | 0.002 |
| | | 14 | 25°C | clear | 5.33 | 2.7 | 0.110 | 0.002 |
| | | 14 | 40°C | clear | 5.25 | 2.5 | 0.313 | 0.004 |

EXAMPLE 8

This example shows the long term stability of the immunoconjugate MF-T-SPDB-DM4 in a preferred lyophilized composition.

The MF-T-SPDB-DM4 conjugate was formulated at approximately 5.0 mg/ml in 10 mM L-Histidine, 130 mM Glycine, 5% sucrose, 0.01% polysorbate 80, pH 5.5. The liquid formulation was lyophilized as described in example 6. The lyophilized composition to be reconstituted to contain 5 mg/ml MF-T-SPDB-DM4 comprises therefore 0.31 mg L-Histidine, 1.95 mg Glycine, 9.99 mg sucrose, and 0.02 mg polysorbate 80 per mg of the immunoconjugate MF-T-SPDB-DM4. Once reconstituted with water, such a lyophilized composition has a pH of about 5.5.

The lyophilized composition was stored for a time period of 24 month at 2 to 8°C. At certain time points (3, 6, 9, 12, 18, and 24 month) samples were reconstituted with sterile water.

After reconstitution (final volume 12.5 ml / vial) the liquid composition was analyzed for usability in pharmaceutical applications. In addition to the assays described above which analyze the physical stability (aggregation, particle formation, etc.) and chemical stability (free DM4 species; DM4/antibody ratio) also the potency as immunoconjugate was analyzed in a cell based bioassay.

As shown in table 8 all relevant parameters are stable for a 24 month time period. All measured parameters including biological potency of the immunoconjugate show that the preferred formulations enable a long shelf live.

**Table 8:** Long term stability (up to 24 months) of immunoconjugate MF-T-SPDB-DM4 in preferred lyophilized formulation. Test results after reconstitution with sterile water.

| Test | Storage Time (months) | | | | | | |
|---|---|---|---|---|---|---|---|
| | **0** | **3** | **6** | **9** | **12** | **18** | **24** |
| Reconstitution time [sec] | 28 | 40 | 39 | 56 | 48 | 46 | 40 |
| Protein conc. [mg/ml] | 4.8 | 5.2 | 4.9 | 4.9 | 4.9 | 5.0 | 4.8 |
| pH-value | 5.6 | 5.5 | 5.6 | 5.5 | 5.6 | 5.5 | 5.7 |
| Visible particles | free | free | free | free | free | free | free |
| HIAC, particles >= 25 µm per vial | 5 | - | - | - | 0 | - | 21 |
| HIAC, particles >= 10 µm per vial | 21 | - | - | - | 15 | - | 9 |

(continued)

| Test | Storage Time (months) | | | | | | |
|---|---|---|---|---|---|---|---|
| | 0 | 3 | 6 | 9 | 12 | 18 | 24 |
| Monomer SEC-HPLC [%] | 92.1 | 91.6 | 92.1 | 92.6 | 92.4 | 90.5 | 90.9 |
| LMW SEC-HPLC [%] | n.d. | n.d. | n.d. | 0.3 | 0.3 | 2.2 | 1.3 |
| Free DM4 species [$\mu$g/mg] | 0.17 | 0.17 | 0.19 | 0.18 | 0.20 | 0.18 | 0.15 |
| DM4/Ab ratio | 3.0 | 3.1 | 3.1 | 3.0 | 3.1 | 3.1 | 3.2 |
| Potency (cell based bioassay) [%] | 55 | 66 | 51 | 69 | 57 | 76 | 50 |

USED METHODS

**Determination of protein concentrations and determination of maytansinoid/antibody ratio (DM4/Ab ratio)**

Protein concentrations and the maytansinoid/antibody ratio (DM4/Ab ratio) were determined via measurement of the absorption at two wavelength (280 nm and 252 nm) using a Nanodrop 2000 (Thermo Scientific). Each solution was measured twice on three independent samples.

Samples were diluted with formulation buffer to obtain a UV-absorption at 280 nm in the range of 0.6 to 1.2. The sample was measured at 252 nm and 280 nm using the formulation buffer as blank. The calculations are shown below.

***Calculations:***

Concentration of DM4 [M]

$$[DM4] = \frac{[(A_{252} \times DF) - (A_{280} \times DF \times AB_{\varepsilon 252/\varepsilon 280})]}{[5323 \times (4,89 - AB_{\varepsilon 252/\varepsilon 280})]}$$

$A_{252}$ = Extinction 252 nm
DF = dilution factor
$A_{280}$ = Extinction 280 nm
$AB_{\varepsilon 252/\varepsilon 280}$ = Ratio of extinction coefficients of antibody 0.35
$AB_{\varepsilon 252}$ = 68614 [M$^{-1}$ (cm)$^{-1}$]
$AB_{\varepsilon 280}$ = 195413 [M$^{-1}$ (cm)$^{-1}$]
$DM4_{\varepsilon 280}$ = 5323 [M$^{-1}$ (cm)$^{-1}$]
$DM4_{\varepsilon 252}$ = 26010 [M$^{-1}$ (cm)$^{-1}$]
$DM4_{\varepsilon 252/\varepsilon 280}$ = Ratio of extinction coefficients of DM4 (4.89)

Concentration of antibody [M]

$$[AB] = \frac{[(A_{280} \times DF) - (5323 \times [conc. of \ DM4])]}{AB_{\varepsilon 280}}$$

$DM4_{\varepsilon 280}$ = 5323 [M$^{-1}$ (cm)$^{-1}$]
$AB_{\varepsilon 280}$ = 195413 [M$^{-1}$ (cm)$^{-1}$]

The DM4/AB ratio

$$= \frac{[DM4]}{[AB]}$$

The concentration of antibody [mg/mL]

$$= [AB] \times MW_{AB}$$

[AB]      = Molar concentration of antibody

$MW_{AB}$   = 146278 g/mol

## Visual inspection

For visual appearance testing solutions containing MF-T-SPDB-DM4 were inspected using a dark background for particles or turbidity. A clear solution after buffer exchange or stress testing is a sign of minor presence or absence of dimer and/or oligomer formation, whereas a visible turbidity of the solution correlates with a high content of dimers and oligomers.

## Dynamic light scattering (DLS)

Dynamic light scattering is a method for analyzing the scattered light generated by a laser. The light scattered by a solubilized or suspended probe can be used to calculate the hydrodynamic radius ($d_H$ [nm]). An increasing hydrodynamic radius is an indication for aggregation of the immunoconjugate. The hydrodynamic radius via DLS was determined using a Horiba LB 550 (Retsch Technology). A measured $d_H$ greater than 25nm was seen as critical. $d_H$ values between 16 nm and 25 nm indicated a well behaved immunoconjugate.

## Differential scanning calorimetry, (DSC)

Protein stability can be measured via determination of the melting temperature. With differential scanning calorimetry (DSC) the melting temperature ($T_m$) of MF-T-SPDB-DM4 in several solutions was measured. Samples were heated from 20°C to 105°C and the melting temperature ($T_m$) was determined using a VP-DSC calorimeter (GE Healthcare).

## Shaking stress test

Immunoconjugates like MF-T-SPDB-DM4 are very sensitive towards mechanical agitation, which take place during production, filling, or transport. Upon a certain intensity of movement the immunoconjugates start to aggregate and to denature. During the shaking stress test liquid compositions containing MF-T-SPDB-DM4 were analyzed for aggregation and denaturation under controlled conditions.

MF-T-SPDB-DM4 containing samples were stressed on a lab shaker (IKA, HS 260) in a temperature controlled chamber (MMM, FrioCell 200). The critical quality parameter aggregation (via visual inspection and DLS) was measured after 24 hours at 20°C and 300 rpm.

## Size exclusion chromatography, SEC

For the determination of monomer and dimer contents as well as the low molecular weight fragments (LMW) and high molecular weight (HMW) contents a size exclusion chromatography (SEC) was performed using an HPLC system. MF-T-SPDB-DM4 was detected using a fluorescence detector and quantified using the area percent method. Standard HPLC SEC columns for proteins were used, e.g. Tosoh Biosep TSK gel G3000 SWXL 5 $\mu$m, 300mm, Length x 7.8mm i.D.

## Detection of non-visible particles HIAC and MFI

For the detection and counting of non-visible particles the HIAC and MFI methods were applied. For MFI (Micro Flow Imaging) measurements a Micro-Flow Imaging™ DPA 4200 system (Brightwell Technologies Inc.) was used in accordance with the supplier's instructions. HIAC measurements were performed using an HIAC 9703+ Liquid Particle Counter (HACH Lange) in combination with a HRLD-150 13-150 $\mu$m sensor in accordance with the supplier's instructions.

## Detection of free maytansinoid with HPLC

The free maytansinoid was determined using an HPLC method. A Supelcosil LC-HISEP, 50 mm x 4.6 mm, 5 $\mu$m (Sigma-Aldrich) or an equivalent column was used in combination with a Zorbax Eclipse XDB-C18, 50 mm x 2.1 mm, 3.5 $\mu$m (Agilent) or an equivalent column. The columns were used with an Agilent HPLC system. The free maytansinoid was separated from protein by HISEP chromatography and subsequently quantified by reversed phase high performance chromatography (RP-HPLC). The columns were used at 35°C. As solvents a mobile phase A (0.1 % TFA in water) and a mobile phase B (0.08 % TFA in acetonitrile) were used. Maytansinoid (DM4) and all other components content is calculated by the Empower calc. routine using linear regression analysis of the rutin hydrate calibration curve in µg/mL. To correct the different correlation factors of rutin hydrate and DM4, the results of DM4 and all other components have to be multiplied by the factor 1.05497.

**Cell based potency assay (bioassay)**

The biological activity of the immunoconjugate MF-T-SPDB-DM4 was tested using a cell based potency assay as described in WO2010/124797 in detail. In brief, mesothelin transfected HT29-cells were seeded into 96-well plates and incubated with a serial dilution of MF-T-SPDB-DM4 over 4 hours. After this incubation with the immunoconjugate the cells were washed carefully with medium and incubated for additional 68 to 96 hours. After this time cellular proliferation was quantified using a standard method (e.g. WST-1 Cell Proliferation Reagent, Roche). The activity ratio compared to a standard value is evaluated and reported.

**Claims**

1.    An immunoconjugate formulation comprising:

    a. 5 mg/ml MF-T-SPDB-DM4, an anti-mesothelin immunoconjugate of a maytansinoid,
    b. 10 mM L-Histidine,
    c. 130 mM Glycine
    d. 5% (w/v) sucrose, and
    e. 0.01% (w/v) polysorbate 80

    wherein the formulation is a buffered aqueous solution having a pH of 5.5.

2.    A lyophilized composition obtained by freeze-drying of a liquid immunoconjugate formulation according to claim 1.

3.    An immunoconjugate formulation obtained by reconstitution of the lyophilized composition of claim 2 in solution.

4.    An immunoconjugate formulation obtained by reconstitution of the lyophilized composition of claim 2 in water.

5.    The lyophilized composition of claim 2 that is to be reconstituted to contain 5 mg/ml MF-T-SPDB-DM4 comprises 0.31 mg L-Histidine, 1.95 mg Glycine, 9.99 mg sucrose, and 0.02 mg polysorbate 80 per mg of the immunoconjugate MF-T-SPDB-DM4. Once reconstituted with water, such a lyophilized composition has a pH of about 5.5.

6.    An immunoconjugate formulation according to any one of the preceding claims for use in a therapeutic application.

**Patentansprüche**

1.    Immunkonjugatformulierung, umfassend:

    a. 5 mg/ml MF-T-SPDB-DM4, ein Anti-Mesothelin-Immunkonjugat eines Maytansinoids,
    b. 10 mM L-Histidin,
    c. 130 mM Glycin
    d. 5% (w/v) Saccharose und
    e. 0,01% (w/v) Polysorbat 80,

    wobei es sich bei der Formulierung um eine gepufferte wässrige Lösung mit einem pH-Wert von 5,5 handelt.

2.    Lyophilisierte Zusammensetzung, erhalten durch Gefriertrocknen einer flüssigen Immunkonjugatformulierung nach Anspruch 1.

3.    Immunkonjugatformulierung, erhalten durch Rekonstitution der lyophilisierten Zusammensetzung nach Anspruch 2 in Lösung.

4.    Immunkonjugatformulierung, erhalten durch Rekonstitution der lyophilisierten Zusammensetzung nach Anspruch 2 in Wasser.

5. Lyophilisierte Zusammensetzung nach Anspruch 2, die so zu rekonstituieren ist, dass sie 5 mg/ml MF-T-SPDB-DM4 enthält und 0,31 mg L-Histidin, 1,95 mg Glycin, 9,99 mg Saccharose und 0,02 mg Polysorbat 80 pro mg des Immunkonjugats MF-T-SPDB-DM4 umfasst. Nach der Rekonstitution mit Wasser weist eine solche lyophilisierte Zusammensetzung einen pH-Wert von etwa 5,5 auf.

6. Immunkonjugatformulierung nach einem der vorhergehenden Ansprüche zur Verwendung in einer therapeutischen Anwendung.


**Revendications**

1. Formulation d'immunoconjugué comprenant :

   a. 5 mg/ml de MF-T-SPDB-DM4, un immunoconjugué anti-mésothéline d'un maytansinoïde,
   b. 10 mM en L-histidine,
   c. 130 mM en glycine,
   d. 5% (poids/volume) de saccharose et
   e. 0,01% (poids/volume) de polysorbate 80,

   la formulation étant une solution aqueuse tamponnée ayant un pH de 5,5.

2. Composition lyophilisée obtenue par lyophilisation d'une formulation liquide d'immunoconjugué selon la revendication 1.

3. Formulation d'immunoconjugué obtenue par reconstitution de la composition lyophilisée selon la revendication 2 en solution.

4. Formulation d'immunoconjugué obtenue par reconstitution de la composition lyophilisée selon la revendication 2 dans de l'eau.

5. Composition lyophilisée selon la revendication 2 à reconstituer pour contenir 5 mg/ml de MF-T-SPDB-DM4, comprenant 0,31 mg de L-histidine, 1,95 mg de glycine, 9,99 mg de saccharose et 0,02 mg de polysorbate 80, par mg d'immunoconjugué MF-T-SPDB-DM4. Une telle composition lyophilisée a un pH d'environ 5,5 dès lors que reconstituée avec de l'eau.

6. Formulation d'immunoconjugué selon l'une quelconque des revendications précédentes destinée à être utilisée dans une application thérapeutique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 931 A
- US 136 A
- WO 2009068204 A
- WO 2010124797 A
- WO 2004004639 A
- WO 2004110498 A
- WO 2007019232 A

**Non-patent literature cited in the description**

- **CHANG, K. ; I. PASTAN.** *Proc. Natl. Acad. Sei. USA,* 1996
- **ARGANI, P. et al.** *Clin. Cancer Res.,* 2001, vol. 7 (12), 3862
- **HASSAN, R.** *Cancer Res.,* 2004, vol. 10, 3937
- **CHARI et al.** *Pharm Res.,* 2003, vol. 20, 1325-1336
- **WANG, W.** *Int. J . Pharm.,* 2000, vol. 203, 1-60